# EUROPEAN PATENT SPECIFICATION

(11) **EP 3 181 125 B1**
(45) Date of publication and mention of the grant of the patent: **19.02.2025**
(21) Application number: 14898418.0
(22) Date of filing: 31.07.2014
(51) Int. Cl.: A61K 9/48, A61J 3/07, A61K 47/14, A61K 47/44

(54) **CAPSULE FORMULATION**
KAPSELFORMULIERUNG
FORMULATION DE CAPSULE

(43) Date of publication of application: 21.06.2017
(73) Proprietor: Capsugel Belgium NV, 2880 Bornem (BE)
(72) Inventor: HUTCHISON, Keith, B-2880 Bornem (BE); TAKUBO, Takahisa, Sagamihara-shi Kanagawa 252-0253 (JP)
(74) Representative: De Clercq & Partners
(86) International application number: PCT/JP2014/070242
(87) International publication number: WO 2016/017006

(56) References cited:
- WO-A1-2011/036601
- WO-A1-2011/036601
- WO-A1-2014/006532
- JP-A- 2010 202 550
- JP-A- H07 196 478
- JP-A- S5 632 413
- US-A1- 2012 134 921
- US-A1- 2012 164 217

## Description

### Technical field

The present invention relates to a capsule formulation.

### Background to the invention

Many active ingredients in healthy food, oral drugs, or the like use a type of formulation that disintegrates in the stomach. Meanwhile, in formulations wherein the efficacy of an active ingredient can be reduced by gastric acid, the active ingredient can pass through the stomach and be delivered to the intestines while being protected from gastric acid, if desired, by applying an acid-resistant coating to the surface of a tableted or capsulated formulation. A method is known for this type of formulation wherein an enteric coating is applied onto a hard capsule membrane (that is, an outer film). However, the enteric coating generally requires additional coating steps following capsule formulation preparation, which is a problem because it increases manufacturing costs. Further, irregularities in coating, and quality stability are also concerns.

Meanwhile, in recent years, hard capsules have been developed and are being sold wherein the hard capsule membranes (outer film) themselves are acid resistant. For example, DRcaps (trademark) made by Capsugel Inc. are hard capsules composed of a hydrophilic-acid resistant capsule membrane. A formulation can become acid resistant just by being encapsulated, and these capsules do not necessarily require further application of an enteric coating on the capsule membrane (patent document 1). These types of acid resistant capsules do not dissolve even when submerged in an acid solution, and are useful for intestinal delivery because the hard capsules do not disintegrate in the stomach of a living organism. Therefore, there is no particular problem if a hard capsule does not disintegrate in the stomach when a sufficiently active ingredient (for example, garlic which can give one garlic breath) is enclosed in a hard capsule as long as the expected effect is obtained.

### Prior art documents

### Patent Documents

Patent Document 1: US Laid-Open Patent No. 2012/0288562A1 relates to acid resistant hard pharmaceutical capsules that meet acid resistance criteria in pharmacopeia and processes for manufacturing such capsules.
Patent Document 2: International Patent Application No. 2014/006532A1 relates to capsule formulations that contain thymoquinone as an active ingredient. The capsule formulations allow storage of thymoquinone for a prolonged period of time at room temperature.
Patent Document 3: US Laid-Open Patent No. 2012/134921A1 describes solid compositions comprising 5-aminolevulinic acid for use in photodynamic diagnosis in the lower part of the gastrointestinal tract. The compositions are stable at room temperature.
Patent Document 4: US Laid-Open Patent No. 2012/164217A1 relates to scented hard capsules that are externally coated with a scenting composition. The scenting composition comprises one of more scenting agents and one or more gliding enhancing agents. The composition is in powder form or oil form at room temperature.
Patent Document 5: International Patent Application No. 2011/036601A1 describes acid resistant hard capsules and aqueous compositions for producing said capsule. The capsules are considered to meet the acid resistance criteria set forth in pharmacopeia.

### Summary of the invention

While studying enteric capsule formulations that include an acid resistant hard capsule, surprisingly the present inventors found that even though the capsule does not disintegrate in the stomach, over time gastric acid gradually enters through the capsule membrane because the capsule membrane contains a hydrophilic material. Therefore, even if a capsule is used wherein the capsule membrane itself is acid resistant, in cases wherein the efficacy of an encapsulated active ingredient is reduced by a small amount of gastric acid (gastric juice), there is a possibility that the active ingredient will deteriorate (changes/be dispersed/be annihilated) when the active ingredient comes in contact with acid (gastric acid), and the expected effect will not be obtained sufficiently.

In the light of the above problems, an object of the present invention is to provide means for protecting active ingredients from gastric acid that seeps into the capsule membrane in capsule formulations including active ingredients wherein components can deteriorate if they come into contact with acid, without necessarily requiring an enteric coating.

### Means to resolve the problems

As a result of earnest study, surprisingly, the present inventors found that the above problems could be resolved by developing a capsule formulation having the following configuration.

That is, the present invention includes the following embodiments:
(1) An enteric capsule formulation comprising an active ingredient and oil, wherein
   said capsule of said formulation comprises a water soluble film forming polymer and gellan gum;
   wherein the weight ratio of said water soluble film forming polymer and said gellan gum in said capsule is 4 to 15 parts by weight of gellan gum relative to 100 parts by weight of said water soluble film forming polymer;
   wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof;
   and said active ingredient is encapsulated in said capsule together with said oil.
(2) The capsule formulation according to (1), wherein said capsule does not have enteric coating.
(3) The capsule formulation according to any one of (1) to (2), wherein said capsule is a hard capsule having a body portion and a cap portion, and the fitted part of said body portion and said cap portion is sealed by band-sealing.
(4) The capsule formulation according to any one of (1) to (3), wherein the amount of said oil in the whole content encapsulated in said capsule is not less than 40% by weight.
(5) The capsule formulation according to any one of (1) to (4), wherein said active ingredient is dispersed or dissolved in said oil.
(6) The capsule formulation according to any one of (1) to ((), wherein a surfactant acceptable for pharmaceuticals or food is further encapsulated in said capsule.
(7) The capsule formulation according to (§), wherein said active ingredient is emulsified in said oil by said surfactant.
(8) The capsule formulation according to any one of (1) to (è), wherein said active ingredient is a pharmaceutical, a dietary supplement, peptides, amino acids, proteins, glycoproteins, enzyme fermented foods, enzymes, coenzymes, vitamins, minerals, living microbes, plant extracts, natural organic matters, or any combination thereof.
(9) A method for producing a capsule formulation comprising an active ingredient and oil,
   wherein said capsule of said formulation is an enteric capsule comprising a water soluble film forming polymer and gellan gum,
   wherein the weight ratio of said water soluble film forming polymer and said gellan gum in said capsule is 4 to 15 parts by weight of gellan gum relative to 100 parts by weight of said water soluble film forming polymer,
   wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof; and said method comprising: dispersing or dissolving said active ingredient in said oil; and encapsulating said oil having the dispersed active ingredient into said capsule.
(10) The method for producing a capsule formulation according to (9), wherein said capsule is a hard capsule have a body portion and a cap portion, and said method comprises sealing the fitted part of said body portion and said cap portion by band-sealing after encapsulation into said capsule.
(11) Use of an oil for protecting active ingredients from gastric acid that seeps into the capsule membrane in enteric capsule formulations including active ingredients,
   wherein said capsule of said formulation comprises a water soluble film forming polymer and gellan gum;
   wherein the weight ratio of said water soluble film forming polymer and said gellan gum in said capsule is 4 to 15 parts by weight of gellan gum relative to 100 parts by weight of said water soluble film forming polymer;
   wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof;
   and said active ingredient is encapsulated in said capsule together with said oil.

### Effect of the invention

The capsule formulation described herein is able to protect an active ingredient that may deteriorate (change/be dispersed/be annihilated) when it comes into contact with acid (gastric acid) seeping into a capsule. That is, even if oil is mixed with acid (gastric acid), due to having separating immiscible properties, acid (gastric acid) seeping into the capsule sinks to the bottom of the capsule because of the difference in the specific gravity of oil without being diffused into an encapsulated oil acceptable for pharmaceuticals or food. Therefore, contact between an acid and an active ingredient can be effectively reduced by separating both the aqueous phase (including acid) and the oil phase (including an active ingredient) within a capsule.

Further, another aspect of the capsule formulation described herein is employing an extremely simple composition, that is, encapsulating an active ingredient dispersed in an oil acceptable for pharmaceuticals or food. Thus, the presently disclosed capsule formulation can easily maintain the subtle balance between protecting against gastric acid and quickly disintegrating in the intestines (for example the small intestine or large intestine), and is advantageous in that it doesn't require an advanced formulation.

### Brief Description of the Figures

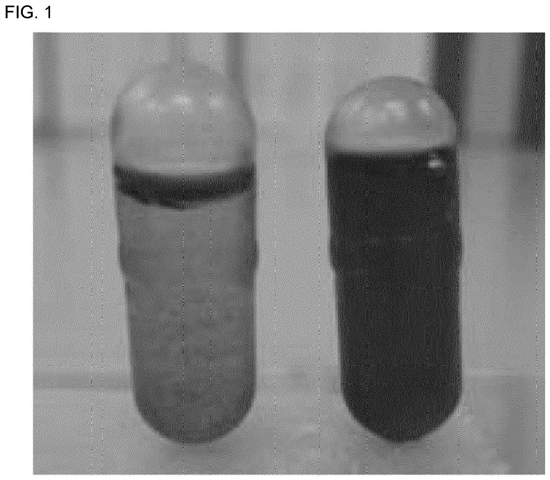

**FIG. 1** is a picture depicting the capsule formulation created in example 1 (left in the picture: MCT (example); right in the picture: glycerin (comparative example)). A litmus reagent was uniformly dispersed in the MCT solution (the liquid in the capsule is a light blue color), and was dissolved in the glycerin solution (the liquid in the capsule is a dark blue color).

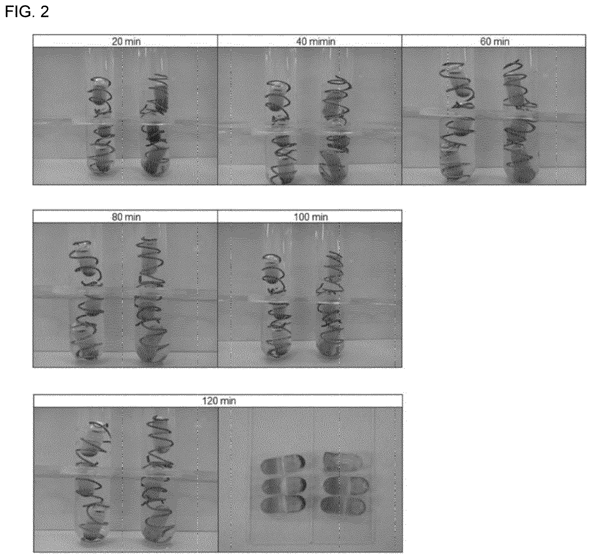

**FIG. 2** is a picture depicting the results of an acid resistance experiment (left side of each picture: MCT (example); right side of each picture: glycerin (comparative example)), wherein the liquid color in a capsule was observed at 20-minute intervals up to 120 minutes after a capsule formulation created by example 1 (the experiment was carried out so that n=3 for both the example and the comparative example) was submerged in the first liquid (pH1.2 liquid) in the Japanese Pharmacopoeia, which was maintained at 37°.

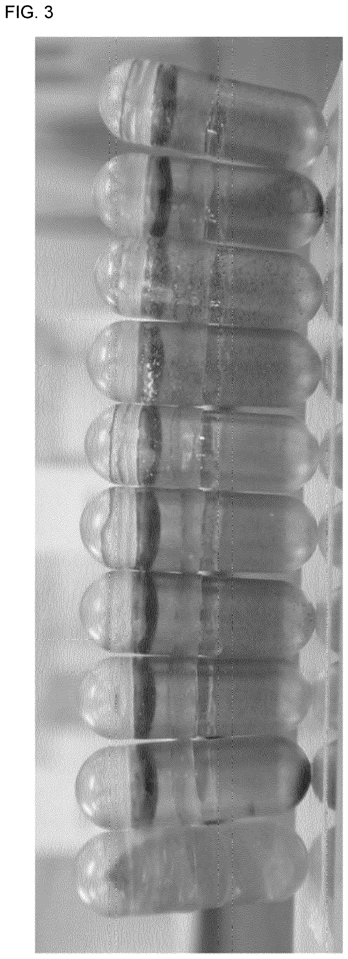

**FIG. 3** is a picture of the capsule formulation created in example 2 prior to being submerged in the first liquid (pH1.2 liquid) in the Japanese Pharmacopoeia, which was maintained at 37° (in order from the bottom of the picture: glycerin fatty acid ester, sorbitan fatty acid ester, safflower oil, olive oil, soybean oil, linseed oil, DHA, rice germ oil, vitamin E, and vitamin A).

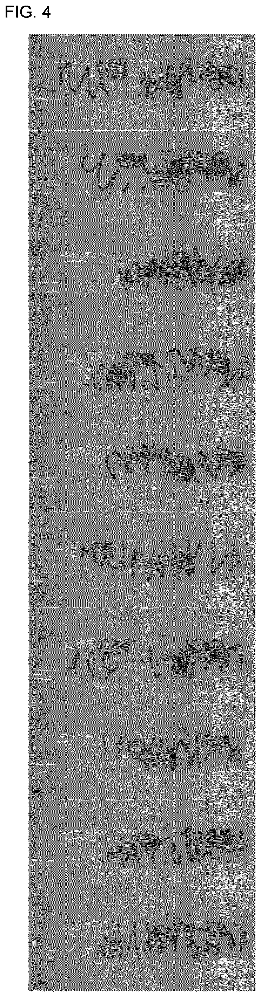

**FIG. 4** is a picture depicting the results of an acid resistance experiment wherein the liquid color in a capsule was observed 120 minutes after the capsule formulation created in example 2 (the experiment was carried out so that n=3 for both the example and the comparative example) was submerged in the first liquid (pH1.2 liquid) in the Japanese Pharmacopoeia, which was maintained at 37° (in order from the bottom of the picture: glycerin fatty acid ester, sorbitan fatty acid ester, safflower oil, olive oil, soybean oil, linseed oil, DHA, rice germ oil, vitamin E, and vitamin A).

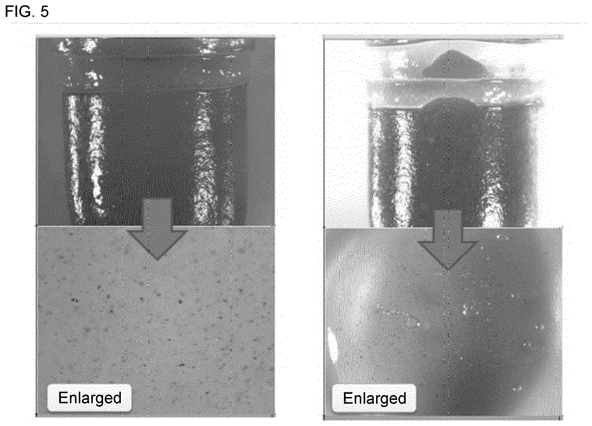

**FIG. 5** depicts pictures taken near the fitted part of a capsule formulation and enlarged pictures thereof after being submerged in the first liquid (pH1.2 liquid) in the Japanese Pharmacopoeia, which was maintained at 37°, in cases wherein both DHA and vitamin E were used (upper left of the picture: DHA, bottom left of the picture: enlarge picture of when DHA was used, upper right of the picture: vitamin E, bottom right of the picture: enlarged picture of when vitamin E was used).

### Embodiments

The present disclosure relates to a capsule formulation containing an active ingredient and oil (also called a capsule formulation related to the present disclosure hereinafter). In the capsule formulation described herein, the capsule is an enteric capsule containing a water soluble film forming polymer and gellan gum, wherein the oil described above is acceptable for pharmaceuticals or foods, and the active ingredient described above is encapsulated in the capsule described above together with the oil described above. Though the capsule formulation may be used for any purpose as long as it is appropriate, it is particularly preferable that the capsule formulation be used as a capsule for pharmaceuticals or dietary supplements.

As long as the capsule can form a membrane that can enclose contents related to the present disclosure (that is, including an active ingredient and oil), the capsule may be either a soft capsule or a hard capsule.

The term "enteric" used herein means that the capsule does not dissolve (or disintegrate) substantively in gastric acid or in the stomach but does dissolve in the intestines (small intestine, large intestine), and is used interchangeably with the term "acid resistance" in the present specification.

Any water soluble film forming polymer known to be used as the base material for a capsule can be used as a water soluble film forming polymer. This type of water soluble film forming polymer can be selected from a group consisting of, but not limited to, for example, a cellulose derivative, preferably hydroxypropylmethylcellulose (HPMC), gelatin, pullulan, or polyvinyl alcohol (PVA), and a starch derivative, preferably hydroxypropylstarch. Preferably, the water soluble film forming polymer is selected from a group consisting of HPMC, gelatin, pullulan, PVA, and hydroxypropylstarch because the water soluble film forming polymer forms a film having optimal mechanistic properties in aspects of the elastic module and brittleness. One type of water soluble film forming polymer may be used or a combination of a plurality of types may be used. More preferably, the film forming polymer contains HPMC and/or gelatin. Alternatively, the film forming polymer is made of HPMC. Yet alternatively, the film forming polymer is made of gelatin.

Gellan gum is an exopolysaccharide produced by fermentation. The ratio of gellan gum to water soluble film forming polymer is not particularly limited as long as the ratio can make the capsule acid resistant, and those skilled in the art can appropriately determine a proper ratio according to the type of water soluble film forming polymer used. For example, gellan gum can be used at a ratio of approximately 4 to 15 parts by weight, wherein approximately 4.5 to 8 parts by weight is preferable, and approximately 4.5 to 6 parts by weight is more preferable relative to 100 parts by weight of water soluble film forming polymer. Alternatively, gellan gum can be used at a ratio of approximately 5 or 5.5 parts by weight relative to 100 parts by weight of water soluble film forming polymer. When less gellan gum is used than the lower limit described above, there is a possibility that the eventual capsule will not be provided sufficient acid resistance against digestive fluids such as gastric acid. Meanwhile, when more gellan gum is used than the upper limit described above, there is a possibility that excess viscosity and excess gelation ability will be generated, making manufacture of high quality capsules difficult.

The capsule described herein can be manufactured by methods already well known among those skilled in the art. For example, when the capsule is a hard capsule, the hard capsule can be manufactured by a method wherein a gel is obtained by submerging a molding pin into an aqueous composition wherein water soluble film forming polymer and gellan gum are dissolved, pulling it out, and drying the aqueous composition adhered to the immersion pin. Plasticizers, preservatives, dispersing reagents (e.g. dextrin, sucrose, mannitol, and maltose), coloring agents, lubricants, disintegrants, surface active agents, and other additives are known, and these can be used appropriately in a capsule described herein.

Commercially available products may be used as capsules, for example, it is preferred that DRcaps (trademark), a hard capsule manufactured by Capsugel, be used.

The capsules may have an enteric coating applied over the membrane of the capsule. However, it is also acceptable if an enteric coating is not applied in order to effectively acquire the advantages described throughout the present specification.

When the capsule is a hard capsule having a body portion and a cap portion covering the body portion, it is preferred that the fitted part (seam) of the body portion configuring the capsule body and the cap portion be sealed using band sealing to prevent the contents from leaking. Preparation of a band sealing solution used for band sealing and sealing methods may be carried out appropriately based on means and techniques that are well known among those skilled in the art. For example, a composition identical to the membrane solution (aqueous composition) of the hard capsule may be used as a band sealing solution. International Application No. PCT/US2013/ 041838 (application date: May 20, 2013) (title of invention "ACID RESISTANT BANDING SOLUTION FOR TWO PIECE HARD CAPSULES"), wherein US Patent Application No. 61/707, 135 (application date: September 28, 2012) forms the basic application, which is an undisclosed application, discloses that an effect wherein further entry of acid is prevented because the internal pressure is driven upward by the volume of acid seeping into the hard capsule as a result of the airtightness of the hard capsule being increased by sealing the fitted part of (the body portion and cap portion of) an acid resistant hard capsule having a 2-piece constitution like the DRcaps (registered trademark) (made by Capsugel) with a band seal and by preventing entry of gastric acid using the fitted part. The band sealing solution disclosed in the US Application may be used for the band sealing solution used for band sealing in the present disclosure, and the composition after the solution thereof is dried may be alcohol: approximately 0% by weight, ammonia water: approximately 0 to 10% by weight, shellac: approximately 20 to 100% by weight, rosin: approximately 0 to 35% by weight, surface active agent: approximately 0 to 10% by weight, dye: approximately 0 to 70% by weight, glycerol, triacetin, polyoxyethylene glycol derivative, polyethylene glycol, or plasticizers such as phthalic acid esters: approximately 0 to 15% by weight, hydrated silicon dioxide, starch, crystalline cellulose, or flow agents such as talc: approximately 0 to 50% by weight.

An active ingredient (can be rephrased active ingredients depending on the case in the present specification) may be, but is not limited to, a pharmaceutical, dietary supplement (for example fucoidan, heme iron, polyphenols, including foods for specified health use or nutritional supplements), peptides or amino acids (for example, royal jelly, ornithine, citrulline, aminolevulinic acid, black vinegar, or hydrophobic amino acids such as, valine, leucine, or isoleucine), proteins (for example lactoproteins such as lactoferrin, collagen, or placenta), glycoproteins, enzyme fermented food (for example nattokinase), coenzymes (for example coenzyme Q10), vitamins (for example β carotene), minerals, living microbes (for example yeasts, lactic acid bacteria, or bifidobacteria), plant extracts (such as crude drugs, herbs, for example, turmeric extract, carrot extract, plum extract, ginkgo leaf extract, blueberry extract, or sweet tea extract), natural organic matter such as propolis, or any combination thereof. Alternatively, a physiologically active substance itself may be indicated in the substances included therein, or a substance that doesn't exhibit a physiological activity itself but can affect delivery of the physiologically active substance to the intestine through deterioration by contact with acid may be indicated. In one aspect, a strongly lipophilic and/or strongly hydrophobic active ingredient is preferred. In another aspect, it is more preferred that an active ingredient have ingredients that deteriorate (for example, change/are decomposed/become annihilated) when they come into contact with acid (gastric acid), for example, wherein the quality or titer thereof decreases. The amount of active ingredient with respect to all the encapsulated content can be included in an amount that can exert an expected degree of effect under given conditions (for example an active ingredient, diluting agent, oil acceptable for pharmaceuticals or foods, capsule, their capacity, or others).

"An oil acceptable for pharmaceuticals or foods" (can be simply rephrased "oil" in the present specification, depending on the case) can reduce or substantively prevent (entirely or partially) an acid (acidic solution, for example, gastric acid in a living organism) from seeping into a capsule through the capsule membrane, and prevent contact with what is encapsulated in the capsule (particularly an active ingredient). That is, the oil described above is not limited, and is not limited particularly if the oil can exist separately without being mixed with the aqueous phase and form an oil phase by exhibiting hydrophobicity with respect to the aqueous phase (includes gastric acid, which is an acidic solution).

An oil as described herein may be a liquid (for example fatty oil) or solid (for example fat) at room temperature. It is preferred that the oil is a liquid at the temperature of a living organism (specifically the temperature of the stomach, around 37°C). In general, fat contains many saturated fatty acids (for example, palmitic acid or stearic acid), while fatty oil contains many unsaturated fatty acids (for example, oleic acid, linoleic acid, or linolenic acid). These may be esterified. It is known that the higher the degree of unsaturation in the oil the lower the melting temperature, and the more likely liquefaction is at low temperature (likely to be oxidized), and surprisingly the present inventors have found that various types of oils are useful as oils in the context of the present disclosure regardless of the degree of unsaturation.

In general, fatty acids with a carbon number of 2 to 4 are called short chain fatty acids (lower fatty acids), fatty acids with a carbon number of 5 to 12 are called medium chain fatty acids, and fatty acids with a carbon number of 12 or more are called long chain fatty acids (higher fatty acids). Since fatty acids with a small carbon number generally have increased hydrophobicity, medium chain fatty acids (for example, medium chain fatty acid triglycerides, for example, tri (caprylic/capric acid) glycerin or tricaprylate glyceryl) and long chain fatty acids are preferred oils in the present context.

Further, it is acceptable if an oil is not a fatty acid, for example fat soluble (oil soluble) substances are also included. For example, vitamin A (oil), vitamin D (oil), vitamin E (oil), vitamin K (oil), and fat soluble vitamins such as derivatives thereof, or glycerophospholipids such as lecithin are also included in the oils described herein. It should be noted that examples of lecithin that can be used include lecithin that can be obtained from plants such as soy, rice, rapeseed, safflower, lecithin that can be obtained from an animal such as an egg yolk, and lecithin derivatives that can be obtained by chemically or enzymatically treating this lecithin. It should be noted that examples of lecithin derivatives include enzymatically decomposed lecithin, hydrogenase-decomposed lecithin, hydroxyl lecithin, phosphatidyl glycerol, phosphatidic acid, and acetylated lecithin.

It is preferred that an oil described herein is a plant oil, animal oil, fish oil, or mineral oil, and edible oil is preferred from the viewpoint of acceptable pharmaceuticals or foods. Further, plant oil is preferred. Examples include safflower oil, olive oil, soybean oil, linseed oil, rice germ oil, wheat germ oil, coconut oil, corn oil, cottonseed oil, palm oil, palm nucleus oil, peanut oil, rapeseed oil, sesame oil, sunflower oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, docosahexaenoic acid (DHA), and eicosapentaenoic acid (EPA), but are not limited thereto.

It is preferable that the specific gravity (density ratio with water as a standard) of oil described herein be 0.96 or lower, wherein 0.955 or lower is more preferable, 0.95 or lower is even more preferable, 0.945 or lower is still more preferable, and 0.94 or lower is still even more preferable. Surprisingly, the present inventors found that the more the specific gravity of oil is separated from that of water, the easier it became to physically separate within the capsule the oil (oil phase) and acid (aqueous phase) seeping into the capsule, and the acid and oil (including for example an active ingredient) mixed less easily, protecting the active ingredient from the acid.

In one aspect, the amount of oil with respect to all the encapsulated content can be included in an amount that can exert an expected degree of effect under given conditions (for example an active ingredient, excipient, capsule, their capacity, or others). For the amount of this type of oil with respect to all the encapsulated content, it is preferred for example that an active ingredient be included in a capsule in an amount that can be sufficiently soaked in oil, and it is more preferred that the active ingredient be dispersed or dissolved in oil. For example, the amount of this type of oil with respect to all the encapsulated content can be 30% by weight or more, 35% by weight or more, 40% by weight or more, or 40 to 99.9% by weight. Those skilled in the art can appropriately determine a suitable amount according to the type of active ingredient and oil, but generally the higher amount of encapsulated oil the more preferred it is.

In one aspect, when an encapsulated active ingredient and other desired components are non-lipophilic, a surfactant acceptable for pharmaceuticals or foods may be further included in the capsule in order to emulsify these components (particularly the active ingredients) in oil. Examples of this type of surfactant include but are not limited to monoglycerol fatty acid esters, diglycerin fatty acid esters, polyglycerol fatty acid esters, sucrose fatty acid esters, sorbitan fatty acid esters, propylene glycol fatty acid esters, or any combination thereof. It is preferred that the amount of this type of surfactant with respect to all encapsulated content be, for example, 5% by weight or less, wherein 3% by weight or less is more preferable, and 1% by weight or less is even more preferable. Further, because the surfactant has amphiphilic properties, lower HLB values (value expressing the degree of affinity a surfactant has for water and oil) are preferred, that is, one that is hydrophobic and/or has strong lipophilicity is preferred. For example, a surfactant with an HLB value of approximately 3 to 6 is relatively preferred because only a part disperses into the aqueous phase, and a surfactant with an HLB value of approximately 1 to 3 is more preferable because it does not disperse into the aqueous phase hardly at all. Those skilled in the art can optimize by appropriately determining an adequate amount of surfactant referencing this technical common sense.

Other than the components described above, an additive such as an excipient, preservative, stabilizer, stabilizing agent, or flavoring agent may be included as long as the effect (that is, protecting an active component from acid) achieved is not inhibited.

In another aspect, the capsule formulation may be manufactured by a method including a step for enclosing an active ingredient; and a step for enclosing an oil acceptable for pharmaceuticals or food in the capsule, and these two steps may be carried out simultaneously or separately. When carrying out these two step simultaneously, a step for dispersing or dissolving the active ingredient in an oil acceptable for pharmaceuticals or food, and a step for enclosing in the capsule oil dispersed or dissolved by the active ingredient may be carried out simultaneously. Further, when using a surfactant, the capsule formulation may be manufactured by a method including a step for mixing an active ingredient and surfactant in an oil acceptable for pharmaceuticals or food and emulsifying the active ingredient in the oil, and a step for enclosing oil emulsified by the active ingredient in the capsule, and these two steps may be carried out simultaneously or separately. When oil is solid at room temperature, for example, one may wait for the active ingredient (and surfactant if desired) to disperse or dissolve into the oil, liquefy the oil by warming it, and use the oil to manufacture a capsule formulation. Specific conditions, procedures, and machinery used in each of these steps may be used appropriately according to the knowledge of those skilled in the art. Further, in order to better produce a capsule, manufacturing a capsule formulation by freely adding while revising various other steps known by those skilled in the art is within the scope of variations expected by those skilled in the art.

The capsule formulation is administered by a method corresponding to the type of active ingredient, target age, gender, patient condition, or other condition. The dose of capsule formulation may be selected appropriately according to usage, target age, gender, patient condition, or other condition. The number of administrations may be selected appropriately according to usage, target age, gender, patient condition, or other condition, for example 3X/day, 2X/day, 1X/day, or a more infrequent number of administrations (for example 1X/week, or 1X/month) may be selected according to the stability thereof in the blood.

Further, the term "includes" used in the present specification is intended to mean that a described item (element, step, member, or number) exists, except for when it is clearly meant to be understood differently given the context, and does not exclude the existence of other items (elements, steps, members, or numbers).

As long as there are no alternate definitions, all of the terms used herein (including technical terms and chemical terms) have the same meaning as widely understood by those skilled in the arts. The terms used herein should be interpreted as having a consistent meaning, the meaning in the present specification and related technical field, as long as an alternate definition is not explicitly stated, and should not be idealized or interpreted using an overly formal meaning.

There are cases wherein the examples may be described with reference to a schematic diagram, and in a case of a schematic diagram, expressions may be overstated in order to clarify the description.

In the present specification, for example when phrased "1 to 10% by weight," those skilled in the art understand that the phrase refers individually and specifically to 1, 2, 3, 4, 5, 6, 7, 8, 9, or 10% by weight.

In the present specification, all values used to express a range of values or component content are interpreted as including the meaning of the term "approximately," as long as it is not explicitly stated otherwise. For example, "10 times" is understood as meaning "approximately 10 times" as long as it's not explicitly stated otherwise.

Below, the present disclosure is described in detail while referencing an example. However, the present disclosure can be realized through various aspects, and should not be interpreted as being limited to the example described herein.

### Examples

### <Example 1>

The fine powder of a litmus reagent that changes to red in response to acid (blue, Wako Pure Chemical Industries Ltd. High grade), used as an active ingredient, is dispersed or dissolved in 0.2% by weight each of MCT, which is an edible oil (medium chain fatty acid triglyceride, coconad MT (made by Kao Co.) and glycerin, which is a water-based edible additive (made by Kao Co.) (dissolves very readily in water, comparative example). Then, 300µL each of this solution is loaded into the separated body portion of an acid resistant hard capsule made by Capsugel (Drcaps (trademark), size no. 2, transparent no color). Then, a capsule formulation is prepared by sealing the fitted part of the cap portion and the body portion of the hard capsule using a band sealing solution (see FIG. 1). It should be noted that the band sealing solution is produced based on the information described in International Application PCT/US2013/041838 (application date May 20, 2013) (title of invention "ACID RESISTANT BANDING SOLUTION FOR TWO PIECE HARD CAPSULES") wherein US Patent Application No. 61/707, 135 (application date: September 28, 2012) forms the basic application, which is an undisclosed application. The composition of the band sealing solution is ethanol: 59.9% by weight, shellac: 37.0% by weight, water: 1.6% by weight, and surfactant (sorbitan fatty acid aster and glycerin fatty acid ester) 1.5% by weight.

These capsule formulations are wrapped in wire to prevent the capsule formulations from floating up, and submerged in the first liquid (pH1.2 liquid) in the Japanese Pharmacopoeia which is maintained at 37° to simulate the gastric acid inside a living organism. Then, the liquid color in the capsule was observed at 20-minute intervals up to 120 minutes (acid resistance experiment) (see FIG. 2).

The results were that in the formulation using glycerin in the comparative example, the entire inside of the capsule turned red after 20 minutes, and it was confirmed that the acid seeping into the capsule had diffused into all of the liquid in the capsule.

Meanwhile, in the formulation using the oil (MCT) from the example, the bottom inside the capsule turned slightly red after 20 minutes, but the color of the liquid in most of the capsule did not change from before being submerged in acid. This is because the oil phase (MCT) and the aqueous phase (seeped acid solution) were separated and did not mix together, though acid seeping into the capsule had accumulated slightly on the bottom of the capsule due to the difference in the specific gravity of oil, and means that the acid solution and litmus reagent separated by oil were isolated. Further, the amount of acid that seeped into the capsule did not increase hardly at all after 120 minutes, and it was confirmed that segregation of the litmus reagent separated by oil and the acid was maintained over a long period of time.

### <Example 2>

The experiment was carried out with the same conditions used in example 1 (amount of oil, measurement conditions, and measurement time), and the effect on acid resistance when another oil or surfactant is enclosed in the capsule was examined. In this example, the following criteria were determined based on the color of the fine litmus powder dissolved or dispersed in oil enclosed in a capsule.
"-": almost all red/unsuitable
"+": about half red/passable
"++": all blue/excellent

The results are shown below.

**Table 1**

| Substance Name | Product Name | Manufacturer Name | Oil Color | Result (color) | Specific Gravity (25°C) | Iodine Value |
|---|---|---|---|---|---|---|
| Glycerin | Glycerin | Kao Co. | None | - | 1.264 | NA |
| Glycerin fatty acid ester | Poem DO-100V | Riken Vitamin Co., Ltd. | None | - | 1.02 | NA |
| Sorbitan fatty acid ester | SO-30V | Nikko Chemicals Co., Ltd. | None | - | 0.95 | NA |
| Safflower oil | Safflower salad oil | Summit Oil Mill Co. Ltd. | None | ++ | 0.92 | 114-150 |
| Olive oil | Pharmacopeia olive oil | Kaneda Co., Ltd. | None | ++ | 0.91 | 70-90 |
| Soybean oil | Soybean oil | Riken Vitamin Co., Ltd. | None | ++ | 0.92 | 114-138 |
| Linseed oil | Linseed oil | Summit Oil Mill Co. | None | ++ | 0.93 | 168-190 |
| DHA | DHA | Nippon Chemical Feed Co., Ltd. | Yellow | + | 0.95 | 188-205 |
| Rice germ oil | Rice germ oil | Tsuno Food Industrial Co., Ltd. | Light yellow | ++ | 0.92 | 92-115 |
| Vitamin E | Vegetable oil E-MIX-1000 containing vitamin E | Tama Biochemical Co., Ltd. | Dark green | ++ | 0.94 | NA |
| Vitamin A | Vitamin A 300 | Riken Vitamin Co., Ltd. | Yellow | ++ | 0.92 | NA |

These capsule formulations were wrapped in wire to prevent the capsule formulations from floating up, and submerged in the first liquid (pH1.2 liquid) in the Japanese Pharmacopoeia maintained at 37° to simulate the gastric acid inside a living organism. The color of the liquid in the capsule 120 minutes after submersion is shown (see FIG. 4).

The results when glycerin fatty acid ester, which is a surfactant replacing oil, and sorbitan fatty acid ester were used were the same as when glycerin was used, the entire inside of the capsule turned red, acidified by the acid seeping into the capsule which had diffused into all of the liquid in the capsule. This shows that when a large amount of hydrophilic surfactant is used, both mix due to the hydrophilic function of the aqueous phase (acid), exposing the active ingredient included in the surfactant to acid.

Meanwhile, when docosahexaenoic acid (DHA) is used, part of the inside of the capsule is acidified. However, when safflower oil, olive oil, soybean oil, linseed oil, rice germ oil, vitamin E, and vitamin A are used, there is no mixing with acid, and the interior of the capsule did not become acidic. Importantly, all oils that obtained a "++" result had a specific gravity of 0.94 or lower. This suggests that the more the specific gravity of oil is separated from that of water, the easier it becomes to physically separate oil from acid seeping into the capsule, and the less easily it is mixed with acid.

It should be noted that because the four oils, DHA, rice germ oil, vitamin E, and vitamin A, are light yellow to dark green, visually confirming the change of the fine litmus powder from blue to red due to acid seeping into oil within the capsule was difficult. Therefore, the color was confirmed by enlarging the litmus particles dispersed within the oil from outside the capsule using a microscope. The determination criteria for this was the same as described above (see FIG. 5).

Further, when indexing stability, surprisingly, the present inventors found that many types of oil expressed acid resistance unrelated to the degree of saturation because the iodine value increases as the degree of saturation for the oil increases (that is, easily oxidizable).

## Claims

1. An enteric capsule formulation comprising an active ingredient and oil, wherein
said capsule of said formulation comprises a water soluble film forming polymer and gellan gum;
wherein the weight ratio of said water soluble film forming polymer and said gellan gum in said capsule is 4 to 15 parts by weight of gellan gum relative to 100 parts by weight of said water soluble film forming polymer;
wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof;
and said active ingredient is encapsulated in said capsule together with said oil.

2. The capsule formulation according to Claim 1, wherein said capsule does not have enteric coating.

3. The capsule formulation according to Claims 1 or 2, wherein said capsule is a hard capsule having a body portion and a cap portion, and the fitted part of said body portion and said cap portion is sealed by band-sealing.

4. The capsule formulation according to any one of Claims 1 to 3, wherein the amount of said oil in the whole content encapsulated in said capsule is not less than 40% by weight.

5. The capsule formulation according to any one of Claims 1 to 4, wherein said active ingredient is dispersed or dissolved in said oil.

6. The capsule formulation according to any one of Claims 1 to 5, wherein a surfactant acceptable for pharmaceuticals or foods is further encapsulated in said capsule.

7. The capsule formulation according to claim 6, wherein said active ingredient is emulsified in said oil by said surfactant.

8. The capsule formulation according to any one of Claims 1 to 7, wherein said active ingredient is a pharmaceutical, a dietary supplement, peptides, amino acids, proteins, glycoproteins, enzyme fermented foods, enzymes, coenzymes, vitamins, minerals, living microbes, plant extracts, natural organic matters, or any combination thereof.

9. A method for producing an enteric capsule formulation comprising an active ingredient and oil, wherein said capsule of said formulation comprises a water soluble film forming polymer and gellan gum,
wherein the weight ratio of said water soluble film forming polymer and said gellan gum in said capsule is 4 to 15 parts by weight of gellan gum relative to 100 parts by weight of said water soluble film forming polymer,
wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof; and said method comprising: dispersing or dissolving said active ingredient in said oil; and encapsulating said oil having the dispersed active ingredient into said capsule.

10. The method for producing a capsule formulation according to Claim 9, wherein said capsule is a hard capsule having a body portion and a cap portion; and said method comprises sealing the fitted part of said body portion and said cap portion by band-sealing after encapsulation into said capsule.

11. Use of an oil for protecting active ingredients from gastric acid that seeps into the capsule membrane in enteric capsule formulations including active ingredients,
wherein said capsule of said formulation comprises a water soluble film forming polymer and gellan gum;
wherein the weight ratio of said water soluble film forming polymer and said gellan gum in said capsule is 4 to 15 parts by weight of gellan gum relative to 100 parts by weight of said water soluble film forming polymer;
wherein said oil is an edible oil, and said edible oil is selected from the group consisting of: medium-chain triglyceride, safflower oil, olive oil, soybean oil, linseed oil, rice germ oil, wheat germ oil, coconut oil, corn oil, cottonseed oil, palm oil, palmnucleus oil, peanut oil, rapeseed oil, sesame oil, sunflower, oil, almond oil, cashew oil, hazelnut oil, macadamia nut oil, mongongo oil, pecan oil, pine nut oil, pistachio oil, walnut oil, calabash seed oil, buffalo gourd oil, pumpkin seed oil, watermelon seed oil, acai berry extract, blackcurrant seed oil, borage seed oil, evening primrose oil, amaranth oil, apricot oil, apple seed oil, argan oil, artichoke oil, avocado oil, babassu oil, ben oil, cape chestnut oil, carob oil, cohune palm oil, coriander oil, dica oil, false flax oil, grape seed oil, hemp oil, kapok seed oil, lallemantia oil, marula oil, meadowfoam seed oil, mustard oil, okra seed oil (hibiscus oil), papaya oil, perilla oil, poppyseed oil, prune kernel oil, quinoa oil, ramtil oil, camellia oil, thistle oil, tomato oil, saw palmetto oil, krill oil, borage oil, vitamin A oil, vitamin D oil, vitamin E oil, vitamin K oil, lecithin, and any combination thereof;
and said active ingredient is encapsulated in said capsule together with said oil.

## Patentansprüche

1. Enterische Kapselformulierung umfassend einen Wirkstoff und Öl, wobei die Kapsel der Formulierung ein wasserlösliches filmbildendes Polymer und Gellangummi umfasst;
wobei das Gewichtsverhältnis des wasserlöslichen filmbildenden Polymers und des Gellangummis in der Kapsel 4 bis 15 Gewichtsteile Gellangummi bezogen auf 100 Gewichtsteile des wasserlöslichen filmbildenden Polymers beträgt;
wobei das Öl ein essbares Öl ist und das essbare Öl ausgewählt ist aus der Gruppe bestehend aus: mittelkettigem Triglycerid, Saflonöl, Olivenöl, Sojabohnenöl, Leinsamenöl, Reiskeimöl, Weizenkeimöl, Kokosnussöl, Maisöl, Baumwollsamenöl, Palmöl, Palmherzöl, Erdnussöl, Rapssamenöl, Sesamöl, Sonnenblumenöl, Mandelöl, Cashewöl, Haselnussöl, Macadamianussöl, Mongongoöl, Pekannussöl, Piniennussöl, Pistazienöl, Walnussöl, Kalebassensamenöl, Büffelkürbisöl, Kürbissamenöl, Wassermelonensamenöl, Acai-Beeren-Extrakt, Johannisbeersamenöl, Borretschsamenöl, Nachtkerzenöl, Amaranthöl, Aprikosenöl, Apfelsamenöl, Arganöl, Artischockenöl, Avocadoöl, Babassuöl, Ben-Öl, Kapkastanienöl, Johannisbrotöl, Cohune-Palmenöl, Korianderöl, Dica-Öl, Leindotteröl, Traubensamenöl, Hanföl, Kapoksamenöl, Lallemantiaöl, Marulaöl, Wiesenschaumkrautsamenöl, Senföl, Okrasamenöl (Hibiskusöl), Papayaöl, Perillaöl, Mohnsamenöl, Pflaumenkernöl, Quinoaöl, Ramtillkrautöl, Kamelienöl, Distelöl, Tomatenöl, Sägepalmenöl, Krillöl, Borretschöl, Vitamin-A-Öl, Vitamin-D-Öl, Vitamin-E-Öl, Vitamin-K-Öl, Lecithin und jeder Kombination davon;
und der Wirkstoff in der Kapsel zusammen mit dem Öl eingekapselt ist.

2. Kapselformulierung nach Anspruch 1, wobei die Kapsel keine enterische Beschichtung aufweist.

3. Kapselformulierung nach Anspruch 1 oder 2, wobei die Kapsel eine Hartkapsel mit einem Körperteil und einem Kappenteil ist und der eingepasste Teil des Körperteils und des Kappenteils durch Bandversiegelung versiegelt ist.

4. Kapselformulierung nach einem der Ansprüche 1 bis 3, wobei die Menge des Öls in dem gesamten in der Kapsel eingekapselten Inhalt nicht weniger als 40 Gew.-% beträgt.

5. Kapselformulierung nach einem der Ansprüche 1 bis 4, wobei der Wirkstoff in dem Öl dispergiert oder aufgelöst ist.

6. Kapselformulierung nach einem der Ansprüche 1 bis 5, wobei ein für Arzneimittel oder Lebensmittel akzeptables Tensid ferner in der Kapsel eingekapselt ist.

7. Kapselformulierung nach Anspruch 6, wobei der Wirkstoff in dem Öl durch das Tensid emulgiert wird.

8. Kapselformulierung nach einem der Ansprüche 1 bis 7, wobei der Wirkstoff ein Arzneimittel, ein Nahrungsergänzungsmittel, Peptide, Aminosäuren, Proteine, Glykoproteine, enzymfermentierte Lebensmittel, Enzyme, Coenzyme, Vitamine, Mineralien, lebende Mikroben, Pflanzenextrakte, natürliche organische Stoffe oder jede Kombination davon ist.

9. Verfahren zur Herstellung einer enterischen Kapselformulierung umfassend einen Wirkstoff und Öl, wobei die Kapsel der Formulierung ein wasserlösliches filmbildendes Polymer und Gellangummi umfasst,
wobei das Gewichtsverhältnis des wasserlöslichen filmbildenden Polymers und des Gellangummis in der Kapsel 4 bis 15 Gewichtsteile Gellangummi bezogen auf 100 Gewichtsteile des wasserlöslichen filmbildenden Polymers ist,
wobei das Öl ein essbares Öl ist und das essbare Öl ausgewählt ist aus der Gruppe bestehend aus: mittelkettigem Triglycerid, Saflonöl, Olivenöl, Sojabohnenöl, Leinsamenöl, Reiskeimöl, Weizenkeimöl, Kokosnussöl, Maisöl, Baumwollsamenöl, Palmöl, Palmherzöl, Erdnussöl, Rapssamenöl, Sesamöl, Sonnenblumenöl, Mandelöl, Cashewöl, Haselnussöl, Macadamianussöl, Mongongoöl, Pekannussöl, Pinienknussöl, Pistazienöl, Walnussöl, Kalebassensamenöl, Büffelkürbisöl, Kürbissamenöl, Wassermelonensamenöl, Acai-Beeren-Extrakt, Johannisbeersamenöl, Borretschsamenöl, Nachtkerzenöl, Amaranthöl, Aprikosenöl, Apfelsamenöl, Arganöl, Artischockenöl, Avocadoöl, Babassuöl, Ben-Öl, Kapkastanienöl, Johannisbrotöl, Cohune-Palmenöl, Korianderöl, Dica-Öl, Leindotteröl, Traubensamenöl, Hanföl, Kapoksamenöl, Lallemantiaöl, Marulaöl, Wiesenschaumkrautsamenöl, Senföl, Okrasamenöl (Hibiskusöl), Papayaöl, Perillaöl, Mohnsamenöl, Pflaumenkernöl, Quinoaöl, Ramtillkrautöl, Kamelienöl, Distelöl, Tomatenöl, Sägepalmenöl, Krillöl, Borretschöl, Vitamin-A-Öl, Vitamin-D-Öl, Vitamin-E-Öl, Vitamin-K-Öl, Lecithin und jede Kombination davon; und das Verfahren umfassend: Dispergieren oder Lösen des Wirkstoffs in dem Öl; und Einkapseln des Öls mit dem dispergierten Wirkstoff in die Kapsel.

10. Verfahren zur Herstellung einer Kapselformulierung nach Anspruch 9, wobei die Kapsel eine Hartkapsel mit einem Körperteil und einem Kappenteil ist; und das Verfahren das Versiegeln des eingepassten Teils des Körperteils und des Kappenteils durch Bandversiegelung nach dem Einkapseln in die Kapsel umfasst.

11. Verwendung eines Öls zum Schutz von Wirkstoffen vor Magensäure, die in die Kapselmembran in enterischen Kapselformulierungen einschließlich Wirkstoffen eindringt, wobei die Kapsel der Formulierung ein wasserlösliches filmbildendes Polymer und Gellangummi umfasst;
wobei das Gewichtsverhältnis des wasserlöslichen filmbildenden Polymers und des Gellangummis in der Kapsel 4 bis 15 Gewichtsteile Gellangummi bezogen auf 100 Gewichtsteile des wasserlöslichen filmbildenden Polymers beträgt;
wobei das Öl ein essbares Öl ist und das essbare Öl ausgewählt ist aus der Gruppe bestehend aus: mittelkettigem Triglycerid, Saflonöl, Olivenöl, Sojabohnenöl, Leinsamenöl, Reiskeimöl, Weizenkeimöl, Kokosnussöl, Maisöl, Baumwollsamenöl, Palmöl, Palmherzöl, Erdnussöl, Rapssamenöl, Sesamöl, Sonnenblumenöl, Mandelöl, Cashewöl, Haselnussöl, Macadamianussöl, Mongongoöl, Pekannussöl, Piniennussöl, Pistazienöl, Walnussöl, Kalebassensamenöl, Büffelkürbisöl, Kürbissamenöl, Wassermelonensamenöl, Acai-Beeren-Extrakt, Johannisbeersamenöl, Borretschsamenöl, Nachtkerzenöl, Amaranthöl, Aprikosenöl, Apfelsamenöl, Arganöl, Artischockenöl, Avocadoöl, Babassuöl, Ben-Öl, Kapkastanienöl, Johannisbrotöl, Cohune-Palmenöl, Korianderöl, Dica-Öl, Leindotteröl, Traubensamenöl, Hanföl, Kapoksamenöl, Lallemantiaöl, Marulaöl, Wiesenschaumkrautsamenöl, Senföl, Okrasamenöl (Hibiskusöl), Papayaöl, Perillaöl, Mohnsamenöl, Pflaumenkernöl, Quinoaöl, Ramtillkrautöl, Kamelienöl, Distelöl, Tomatenöl, Sägepalmenöl, Krillöl, Borretschöl, Vitamin-A-Öl, Vitamin-D-Öl, Vitamin-E-Öl, Vitamin-K-Öl, Lecithin und jede Kombination davon;
und der Wirkstoff in der Kapsel zusammen mit dem Öl eingekapselt ist.

## Revendications

1. Formulation de capsule entérique comprenant un ingrédient actif et de l'huile, dans laquelle ladite capsule de ladite formulation comprend un polymère filmogène soluble dans l'eau et de la gomme gellane ;
dans laquelle le rapport pondéral dudit polymère filmogène soluble dans l'eau et de ladite gomme gellane dans ladite capsule est de 4 à 15 parties en poids de gomme gellane par rapport à 100 parties en poids dudit polymère filmogène soluble dans l'eau ;
dans laquelle ladite huile est une huile comestible, et ladite huile comestible est choisie dans le groupe constitué de : triglycéride à chaîne moyenne, huile de carthame, huile d'olive, huile de soja, huile de graine de lin, huile de germe de riz, huile de germe de blé, huile de noix de coco, huile de maïs, huile de graine de coton, huile de palme, huile de coeur de palmier, huile d'arachide, huile de graine de colza, huile de sésame, huile de tournesol, huile d'amande, huile de noix de cajou, huile de noisette, huile de noix de macadamia, huile de mongongo, huile de noix de pécan, huile de pignon de pin, huile de pistache, huile de noyer, huile de graine de calebasse, huile de courge buffalo, huile de graine de citrouille, huile de graine de pastèque, extrait de baie d'açaï, huile de graine de cassis, huile de graine de bourrache, huile d'onagre, huile d'amarante, huile d'abricot, huile de graine de pomme, huile d'argan, huile d'artichaut, huile d'avocat, huile de babassu, huile de ben, huile de châtaigne du Cap, huile de caroube, huile de palme cohune, huile de coriandre, huile de dica, huile de faux lin, huile de graine de raisin, huile de chanvre, huile de graine de kapok, huile de lallemantia, huile de marula, huile de graine de limnanthe, huile de moutarde, huile de graine de gombo (huile d'hibiscus), huile de papaye, huile de périlla, huile de graine de pavot, huile de noyau de pruneau, huile de quinoa, huile de ramtil, huile de camélia, huile de chardon, huile de tomate, huile de palmier nain, huile de krill, huile de bourrache, huile de vitamine A, huile de vitamine D, huile de vitamine E, huile de vitamine K, lécithine et toute combinaison de celles-ci ;
et ledit ingrédient actif est encapsulé dans ladite capsule avec ladite huile.

2. Formulation de capsule selon la revendication 1, dans laquelle ladite capsule n'a pas d'enrobage entérique.

3. Formulation de capsule selon les revendications 1 ou 2, dans laquelle ladite capsule est une capsule dure ayant une partie corps et une partie capuchon, et la partie ajustée de ladite partie corps et de ladite partie capuchon est scellée par scellement par bande.

4. Formulation de capsule selon l'une quelconque des revendications 1 à 3, dans laquelle la quantité de ladite huile dans l'ensemble du contenu encapsulé dans ladite capsule n'est pas inférieure à 40 % en poids.

5. Formulation de capsule selon l'une quelconque des revendications 1 à 4, dans laquelle ledit ingrédient actif est dispersé ou dissous dans ladite huile.

6. Formulation de capsule selon l'une quelconque des revendications 1 à 5, dans laquelle un tensioactif acceptable pour les produits pharmaceutiques ou les aliments est en outre encapsulé dans ladite capsule.

7. Formulation de capsule selon la revendication 6, dans laquelle ledit ingrédient actif est émulsifié dans ladite huile par ledit tensioactif.

8. Formulation de capsule selon l'une quelconque des revendications 1 à 7, dans laquelle ledit ingrédient actif est un produit pharmaceutique, un supplément diététique, des peptides, des acides aminés, des protéines, des glycoprotéines, des aliments fermentés par des enzymes, des enzymes, des coenzymes, des vitamines, des minéraux, des microbes vivants, des extraits de plantes, des matières organiques naturelles ou toute combinaison de ceux-ci.

9. Procédé de production d'une formulation de capsule entérique comprenant un ingrédient actif et de l'huile, dans lequel ladite capsule de ladite formulation comprend un polymère filmogène soluble dans l'eau et de la gomme gellane,
dans lequel le rapport pondéral dudit polymère filmogène soluble dans l'eau et de ladite gomme gellane dans ladite capsule est de 4 à 15 parties en poids de gomme gellane par rapport à 100 parties en poids dudit polymère filmogène soluble dans l'eau,
dans lequel ladite huile est une huile comestible, et ladite huile comestible est choisie dans le groupe constitué de : triglycéride à chaîne moyenne, huile de carthame, huile d'olive, huile de soja, huile de graine de lin, huile de germe de riz, huile de germe de blé, huile de noix de coco, huile de maïs, huile de graine de coton, huile de palme, huile de coeur de palmier, huile d'arachide, huile de graine de colza, huile de sésame, huile de tournesol, huile d'amande, huile de noix de cajou, huile de noisette, huile de noix de macadamia, huile de mongongo, huile de noix de pécan, huile de pignon de pin, huile de pistache, huile de noyer, huile de graine de calebasse, huile de courge buffalo, huile de graine de citrouille, huile de graine de pastèque, extrait de baie d'açaï, huile de graine de cassis, huile de graine de bourrache, huile d'onagre, huile d'amarante, huile d'abricot, huile de graine de pomme, huile d'argan, huile d'artichaut, huile d'avocat, huile de babassu, huile de ben, huile de châtaigne du Cap, huile de caroube, huile de palmiste cohune, huile de coriandre, huile de dica, huile de faux lin, huile de graine de raisin, huile de chanvre, huile de graine de kapok, huile de lallemantia, huile de marula, huile de graine de limnanthe, huile de moutarde, huile de graine de gombo (huile d'hibiscus), huile de papaye, huile de périlla, huile de graine de pavot, huile de noyau de pruneau, huile de quinoa, huile de ramtil, huile de camélia, huile de chardon, huile de tomate, huile de palmier nain, huile de krill, huile de bourrache, huile de vitamine A, huile de vitamine D, huile de vitamine E, huile de vitamine K, lécithine, et toute combinaison de celles-ci ; et ledit procédé comprenant : la dispersion ou la dissolution dudit ingrédient actif dans ladite huile ; et l'encapsulation de ladite huile ayant l'ingrédient actif dispersé dans ladite capsule.

10. Procédé de production d'une formulation de capsule selon la revendication 9, dans lequel ladite capsule est une capsule dure ayant une partie corps et une partie capuchon ; et ledit procédé comprend le scellement de la partie ajustée de ladite partie corps et de ladite partie capuchon par scellement par bande après encapsulation dans ladite capsule.

11. Utilisation d'une huile pour protéger les ingrédients actifs de l'acide gastrique qui s'infiltre dans la membrane de capsule dans des formulations de capsules entériques comportant des ingrédients actifs, dans laquelle ladite capsule de ladite formulation comprend un polymère filmogène soluble dans l'eau et de la gomme gellane ;
dans laquelle le rapport pondéral dudit polymère filmogène soluble dans l'eau et de ladite gomme gellane dans ladite capsule est de 4 à 15 parties en poids de gomme gellane par rapport à 100 parties en poids dudit polymère filmogène soluble dans l'eau ;
dans laquelle ladite huile est une huile comestible, et ladite huile comestible est choisie dans le groupe constitué de : triglycéride à chaîne moyenne, huile de carthame, huile d'olive, huile de soja, huile de graine de lin, huile de germe de riz, huile de germe de blé, huile de noix de coco, huile de maïs, huile de graine de coton, huile de palme, huile de coeur de palmier, huile d'arachide, huile de graine de colza, huile de sésame, huile de tournesol, huile d'amande, huile de noix de cajou, huile de noisette, huile de noix de macadamia, huile de mongongo, huile de noix de pécan, huile de pignon de pin, huile de pistache, huile de noyer, huile de graine de calebasse, huile de courge buffalo, huile de graine de citrouille, huile de graine de pastèque, extrait de baie d'açaï, huile de graine de cassis, huile de graine de bourrache, huile d'onagre, huile d'amarante, huile d'abricot, huile de graine de pomme, huile d'argan, huile d'artichaut, huile d'avocat, huile de babassu, huile de ben, huile de châtaigne du Cap, huile de caroube, huile de palme cohune, huile de coriandre, huile de dica, huile de faux lin, huile de graine de raisin, huile de chanvre, huile de graine de kapok, huile de lallemantia, huile de marula, huile de graine de limnanthe, huile de moutarde, huile de graine de gombo (huile d'hibiscus), huile de papaye, huile de périlla, huile de graine de pavot, huile de noyau de pruneau, huile de quinoa, huile de ramtil, huile de camélia, huile de chardon, huile de tomate, huile de palmier nain, huile de krill, huile de bourrache, huile de vitamine A, huile de vitamine D, huile de vitamine E, huile de vitamine K, lécithine et toute combinaison de celles-ci ;
et ledit ingrédient actif est encapsulé dans ladite capsule avec ladite huile.
